# EUROPÄISCHE PATENTANMELDUNG

(11) **EP 2 477 168 A1**
(43) Veröffentlichungstag der Anmeldung: **18.07.2012**
(21) Anmeldenummer: 12151221.4
(22) Anmeldetag: 16.01.2012
(51) Int. Cl.: G08B 21/24

(54) **System zur Hygienekontrolle, Sensoreinrichtung hierfür, sowie Verfahren zur Hygienekontrolle**

(30) Priorität: 14.01.2011 DE 102011009240
(71) Anmelder: Hyb D.O.O., 8310 Sentjernej (SI)
(72) Erfinder: Pavlin, Marko, 8000 Novo Mesto (SI)
(74) Vertreter: Dreiss

(57) **Zusammenfassung**

System zur Unterstützung der Reinhaltung eines von Personen betretbaren Hygienebereiches, mit einer Reinigungseinrichtung, die zur Reinigung einer Hand einer Person betätigbar ist, mit einer Warneinrichtung zum Auslösen eines Warnsignals, mit einem Näherungssensor zur Detektion eines Eintritts einer Person in den Hygienebereich oder eines Austritts einer Person aus dem Hygienebereich, wobei die Warneinrichtung derart mit dem Näherungssensor zusammenwirkt, dass das Warnsignal bei Detektion eines Eintritts oder Austritts auslösbar ist, und wobei die Warneinrichtung derart mit der Reinigungseinrichtung zusammenwirkt, dass die Auslösung des Warnsignals durch die Betätigung der Reinigungseinrichtung unterdrückbar ist. Die Erfindung betrifft außerdem eine Sensoreinrichtung zur Verwendung mit einem derartigen System, sowie ein Verfahren zur Unterstützung der Reinhaltung eines von Personen betretbaren Hygienebereiches.

## Beschreibung

Die Erfindung betrifft ein System zur Unterstützung beziehungsweise zur Kontrolle der Reinhaltung eines von Personen betretbaren Hygienebereiches. Außerdem betrifft die Erfindung eine Sensoreinrichtung zur Verwendung mit einem solchen System, sowie ein Verfahren zur Unterstützung beziehungsweise Kontrolle der Reinhaltung eines von Personen betretbaren Hygienebereiches.

Solche Systeme finden beispielsweise in Krankenhäusern Verwendung. Hier ist oftmals in bestimmten Bereichen, im Folgenden Hygienebereiche genannt, ein hohes Maß an Sauberkeit zu wahren beziehungsweise eine Kontamination mit Krankheitserregern zu verhindern. Solche Hygienebereiche können beispielsweise ein Krankenbett umfassen, z.B. für einen infektionsgefährdeten Patienten. Denkbar ist jedoch auch, dass ganze Abschnitte oder Bereiche eines Raumes oder ganze Räume, wie beispielsweise ein OP-Saal oder ein Quarantänebereich einer Station, einen Hygienebereich bilden sollen. Wesentliche Ursache für eine Kontamination eines solchen Hygienebereiches ist unzureichendes oder unterbleibendes Händewaschen von Personen, insbesondere medizinischem Personal.

Zur Kontrolle der Handhygiene sind verschiedene Ansätze bekannt.

In der GB 2 446 871 A ist beispielsweise ein System beschrieben, welches die Durchführung einer Reinigungsprozedur registriert und bei unterbliebener Reinigungsprozedur die Bewegungsfreiheit einer im Wirkungsbereich des Systems befindlichen Person einschränkt, beispielsweise durch Verschließen einer Tür.

Die GB 2 417 811 A zeigt einen Handwasch-Detektor, der von einer Person am Handgelenk getragen wird und Zeitabstände zwischen den von der Person durchgeführten Handwasch-Vorgängen registriert. Wird zwischen diesen eine vorgegebene Zeitdauer überschritten, so kann ein Alarm ausgelöst werden.
In der US 2009 0219131 A1 ist ein System beschrieben, bei dem eine Vielzahl von Reinigungsmittelspendern, welche jeweils Sensoreinrichtungen aufweisen, mit einem Computernetzwerk verbunden sind. Die Reinigungsmittelspender registrieren, ob eine Betätigung zur Reinigung stattgefunden hat und auch die Identität der betätigenden Person. Diese Daten werden über das Computernetzwerk zu einem Server übertragen und mit anderen Daten, wie beispielsweise Zeitpunkt des Reinigungsvorganges, beispielsweise als Teil einer Patientenakte, gespeichert.

Der Erfindung liegt die Aufgabe zugrunde, die Reinhaltung eines von Personen betretbaren Hygienebereiches zuverlässig in einer Art und Weise zu unterstützen, die flexibel an verschiedene Bedingungen, wie beispielsweise räumliche Gegebenheiten oder durchzuführende Arbeitsabläufe, anpassbar ist.

Diese Aufgabe wird von einem System bzw. Vorrichtung zur Unterstützung der Reinhaltung eines von Personen betretbaren Hygienebereiches gemäß dem Anspruch 1 gelöst. Darüber hinaus wird die Aufgabe von einer Sensoreinrichtung gemäß Anspruch 17 sowie einem Verfahren zur Unterstützung der Reinhaltung eines von Personen betretbaren Hygienebereiches gemäß Anspruch 19 gelöst.

Das lösungsgemäße System bzw. die Vorrichtung weist eine Reinigungseinrichtung auf, welche zur Reinigung einer Hand einer Person betätigbar ist. Außerdem ist eine Warneinrichtung zum Auslösen eines Warnsignals vorgesehen, sowie ein Näherungssensor zur Detektion eines Eintritts einer Person in den Hygienebereich oder eines Austritts einer Person aus dem Hygienebereich. Dabei wirkt die Warneinrichtung derart mit dem Näherungssensor zusammen, dass das Warnsignal bei Detektion, das heißt bei Erkennung, eines Eintritts oder Austritts auslösbar ist. Dabei wirkt die Warneinrichtung außerdem derart mit der Reinigungseinrichtung zusammenwirkt, dass die Auslösung des Warnsignals durch die Betätigung der Reinigungseinrichtung unterdrückbar ist.

Mit Hilfe dieses Systems kann eine Person, die den Hygienebereich betritt oder verlässt, dazu angehalten werden, erforderliche Hygienemaßnahmen durchzuführen, insbesondere die Hände zu waschen. Folglich wird eine der wesentlichen Ursachen für die Kontamination eines Hygienebereiches unterbunden. Insofern wird durch das lösungsgemäße System insbesondere ein System zur Überwachung der Handhygiene bzw. zur Handwaschkontrolle bereitgestellt.

Das lösungsgemäße System arbeitet zuverlässig und ist flexibel anpassbar, da beispielsweise der Näherungssensor den räumlichen Gegebenheiten entsprechend angepasst und angeordnet werden kann. Anders als bei bekannten Systemen wird insbesondere das medizinische Personal im Klinikalltag bei seiner Arbeit nicht störend behindert, da keine aufwändigen Kontrollprozeduren durchlaufen werden müssen. Außerdem kann das System komfortabel in die bestehende Klinikinfrastruktur eingegliedert werden. Dies ist grundsätzlich ohne aufwändige Umbaumaßnahmen möglich. Dies alles trägt dazu bei, dass das erfindungsgemäße System beim Krankenhauspersonal eine hohe Akzeptanz finden kann, was letztlich seiner Wirksamkeit zugute kommt und die Überwachungssicherheit erhöht.

Der Hygienebereich, insbesondere seine Ausgestaltung und seine Größe, wird unter anderem durch die Reichweite des Näherungssensors beeinflusst. Insbesondere kann der Hygienebereich ein Krankenbett umfassen, welches vor Verunreinigungen oder Kontamination mit Krankheitserregern bewahrt werden soll. Denkbar ist aber auch, dass das System an einer Zugangstür oder an einem Durchgang zu einem Raum mit erhöhten Hygieneanforderungen angeordnet ist. In diesem Fall wird der Hygienebereich von dem so überwachten Raum gebildet bzw. umfasst diesen. Durch entsprechende Auswahl, Ausgestaltung und Anordnung des Näherungssensors kann das erfindungsgemäße System folglich an verschiedene Kontrollsituationen angepasst werden. Das genannte System ist dabei nicht eingeschränkt auf die Verwendung eines Näherungssensors. Vielmehr können auch mehrere Näherungssensoren in der erfindungsgemäßen Art und Weise angeordnet sein und mit den übrigen Teilen des Systems, insbesondere der Warneinrichtung, zusammenwirken.
Unter einer Reinigungseinrichtung ist in diesem Zusammenhang ein Mittel oder eine Vorrichtung zum Reinigen im weiten Sinne zu verstehen. Der Begriff Reinigen umfasst hier beispielsweise Waschen und/oder Entfernen von Verunreinigungen und/oder auch Abtöten beziehungsweise Unschädlichmachen von Krankheitserregern. Sinn ist allgemein die Verhinderung von Verschmutzung und/oder Kontamination des Hygienebereiches. Neben der Reinigung der Hände der betätigenden Person kommt insofern auch die Reinigung von mitgeführten Gerätschaften, beispielsweise Handschuhen oder Untersuchungsgeräte, in Betracht.

Als Reinigungseinrichtung können insbesondere übliche, bereits in der Klinik-Infrastruktur vorhandene Handwaschbecken, Seifenspender und/oder Desinfektionsmittelspender, zum Einsatz kommen. Das erfindungsgemäße System lässt sich somit kostengünstig in die vorhandene Krankenhausausstattung integrieren.

Ein Warnsignal wird dann ausgelöst, wenn der Eintritt einer Person in beziehungsweise der Austritt einer Person aus dem Hygienebereich von dem Näherungssensor detektiert wird, ohne dass ein Reinigungsschritt durch Betätigung der Reinigungseinrichtung durchgeführt wurde. Die Auslösung des Warnsignals unterbleibt, wenn die Reinigungseinrichtung betätigt wird. Hat keine Auslösung stattgefunden, so wird kein Warnsignal abgegeben.

Vorzugsweise ist in diesem Zusammenhang vorgesehen, dass mit der Auslösung ein Warnsignal startet, welches über eine Warndauer anhält. Dies gilt selbstverständlich nur dann, sofern überhaupt eine Auslösung des Warnsignals stattgefunden hat, das heißt sofern die Auslösung nicht unterdrückt wurde. Die Warndauer wird beispielsweise im Bereich von zehn Sekunden bis dreißig Sekunden gewählt. Auch ein Daueralarm ist denkbar.

Eine vorteilhafte Ausgestaltung ergibt sich außerdem dadurch, dass die Reinigungseinrichtung derart mit dem Näherungssensor zusammenwirkt, dass nach Auslösung eines Warnsignals das anhaltende Warnsignal unterdrückt wird, wenn eine Betätigung der Reinigungseinrichtung erfolgt. Dadurch kann eine Person, die nicht den bestimmungsgemäßen Reinigungsvorgang durchgeführt hat, zum Nachholen des Reinigungsvorgangs veranlasst werden. Betätigt die Person doch noch die Reinigungseinrichtung, so wird das Warnsignal abgeschaltet und eine weitere Störung vermieden.

Zur vorteilhaften Ausgestaltung des Systems ist die Reinigungseinrichtung und/oder der Näherungssensor innerhalb des Hygienebereiches angeordnet. Dies hat den Vorteil, dass durch das System auch eine Verunreinigung beziehungsweise Kontamination der genannten Bestandteile des Systems verhindert wird. Denkbar ist jedoch auch, dass die Reinigungseinrichtung und/oder der Näherungssensor außerhalb des Hygienebereiches angeordnet ist. Eine solche Anordnung kann wünschenswert sein, wenn im Hygienebereich Gegenstände beispielsweise aus Platzgründen unerwünscht sind.

Zur weiteren Ausgestaltung der Erfindung ist vorgesehen, dass die Warneinrichtung derart mit dem Näherungssensor zusammenwirkt, dass das Warnsignal bei Detektion eines Eintritts einer Person in den Hygienebereich oder eines Austritts einer Person aus dem Hygienebereich erst nach einer Wartedauer nach der Detektion auslösbar ist. Die Wartedauer beschreibt also ein zusammenhängendes Zeitintervall, dass sich an die Detektion anschließt, wobei ein Warnsignal erst nach Ablauf dieses Zeitintervalls auslösbar ist. Die Wartedauer liegt insbesondere im Bereich von zehn Sekunden bis dreißig Sekunden. Das Vorsehen der Wartedauer ist insbesondere vorteilhaft, wenn die Reinigungseinrichtung im Hygienebereich angeordnet ist. In diesem Fall wird eine Person bereits detektiert, wenn sie sich der Reinigungseinrichtung zum Reinigen der Hände nähert. Erfolgt innerhalb der Wartedauer nach Detektion eine Betätigung der Reinigungseinrichtung, so wird dennoch kein Warnsignal ausgelöst.

Vorteilhaft ist es, wenn die Reinigungseinrichtung derart mit der Warneinrichtung zusammenwirkt, dass aufgrund einer Betätigung der Reinigungseinrichtung die Auslösung des Warnsignals unmittelbar unterdrückt wird. Insbesondere ist denkbar, dass die Auslösung des Warnsignals durch Betätigung der Reinigungseinrichtung immer unterdrückt wird. Insofern erfolgt die Überwachung ausschließlich hinsichtlich eines Eintritts in den Hygienebereich und darauf, ob in unmittelbarem zeitlichen Zusammenhang die Reinigungseinrichtung betätigt wurde. Eine Überwachung der Personen selbst erfolgt nicht. Es wird nicht personenbezogen erfasst, ob für eine bestimmte Person ein Reinigungsvorgang erfolgt ist. Dies kann beispielsweise aus Gründen der Datensicherheit oder zur Wahrung der Privatsphäre von Personen erwünscht sein.

Eine vorteilhafte Ausgestaltung ergibt sich auch dadurch, dass die Warneinrichtung derart mit der Reinigungseinrichtung zusammenwirkt, dass innerhalb einer Durchgangszeitdauer nach Betätigung der Reinigungseinrichtung die Auslösung des Warnsignals unterbleibt.

Die Durchgangszeitdauer beschreibt dabei ein zusammenhängendes Zeitintervall, das sich an die Betätigung anschließt, wobei während der Durchgangsdauer ein Eintritt einer Person in den Hygienebereich beziehungsweise ein Austritt einer Person aus dem Hygienebereich ohne Auslösung eines Warnsignals möglich ist. Die Auslösung unterbleibt also, wenn die Detektion (die ansonsten ein Warnsignal auslösen würde) innerhalb der Durchgangszeitdauer nach der Betätigung erfolgt. Insofern ist vorgesehen, dass die Reinigungseinrichtung derart mit der Warneinrichtung zusammenwirkt, dass die Auslösung des Warnsignals durch Betätigung der Reinigungseinrichtung für eine Durchgangszeitdauer nach der Betätigung unterdrückt wird. Wenn keine Auslösung erfolgt, so wird in der Folge auch kein Warnsignal abgegeben.

Ist beispielsweise die Reinigungseinrichtung außerhalb des Hygienebereiches angeordnet, wird durch das Vorsehen der Durchgangszeitdauer ermöglicht, dass eine Person nach Durchführung eines Reinigungsschrittes in den Hygienebereich einzutreten kann.

Solange eine in den Hygienebereich eingetretene Person in dem Hygienebereich bleibt und somit kein Eintritt beziehungsweise Austritt detektiert wird, findet keine Auslösung statt.

Bevorzugt ist außerdem eine Ausgestaltung des Systems, bei der eine Warnunterdrückungseinrichtung vorgesehen ist, die zur Unterdrückung der Auslösung des Warnsignals unabhängig von der Betätigung der Reinigungseinrichtung betätigbar ist. Die Betätigung der Warnunterdrückungseinrichtung ermöglicht es somit, den Hygienebereich in bestimmten Fällen ohne Betätigung der Reinigungseinrichtung zu betreten, ohne dass ein Warnsignal ausgelöst wird. Dies kann beispielsweise erwünscht sein, wenn im konkreten Fall keine Verunreinigung des Hygienebereichs droht, beispielsweise weil ein bloßer Durchgang durch den Hygienebereich oder lediglich eine Sichtkontrolle eines im Hygienebereich liegenden Patienten oder medizinischer Geräte beabsichtigt ist. Die genannte Ausgestaltung ermöglicht es in diesem Fall, die Betätigung der Reinigungseinrichtung und beispielsweise Verbrauch von Reinigungsmittel einzusparen.

Vorteilhaft ist, wenn die Warnunterdrückungseinrichtung derart mit der Warneinrichtung zusammenwirkt, dass die Auslösung des Warnsignals nach Betätigung der Warnunterdrückungseinrichtung für eine Stummzeitdauer unterdrückbar ist. Es ist also vorgesehen, dass innerhalb einer Stummzeitdauer nach Betätigung der Warnunterdrückungseinrichtung die Auslösung des Warnsignals unterbleibt. Ein Warnsignal wird also nicht ausgelöst, wenn die Detektion eines Eintritts einer Person in den Hygienebereich beziehungsweise eines Austritts einer Person aus dem Hygienebereich innerhalb der Stummzeitdauer nach der Betätigung der Warnunterdrückungseinrichtung erfolgt.

Es ist außerdem denkbar, dass die
Warnunterdrückungseinrichtung derart mit dem Näherungssensor zusammenwirkt, dass die Detektion des Eintritts einer Person in den Hygienebereich oder des Austritts einer Person aus dem Hygienebereich durch die Betätigung der
Warnunterdrückungseinrichtung für eine Stummzeitdauer unterdrückbar ist. In diesem Fall wird also bereits die Detektion unterdrückt, die zur Auslösung eines Warnsignals führen könnte.

Die Warnunterdrückungseinrichtung ist insbesondere als Druckschalter oder Sensorfeld ausgebildet und
vorteilhafterweise im Bereich der Reinigungseinrichtung angeordnet. Dadurch kann die Warnunterdrückungseinrichtung alternativ zur Reinigungseinrichtung betätigt werden, ohne dass ein anderer Ort aufgesucht werden muss.

In anderen Situationen kann jedoch vorteilhaft sein, wenn die Warnunterdrückungseinrichtung von der Reinigungseinrichtung beabstandet, beispielsweise im Bereich eines weiteren Zugangsbereichs zum Hygienebereich oder eines Durchgangsbereichs des Hygienebereichs, angeordnet ist. Eine Person muss sich dann nicht zur Reinigungseinrichtung begeben, um einen Eintritt in beziehungsweise einen Austritt aus dem Hygienebereich ohne Auslösung des Warnsignals zu ermöglichen.

Zur weiteren Ausgestaltung des Systems ist der Näherungssensor als Kapazitätssensor oder als optischer Sensor oder als Infrarotsensor zur Detektion einer Person ausgebildet. Mit derartigen Sensoren wird die Person aufgrund der physikalischen Eigenschaften des menschlichen Körpers, beispielsweise die dielektrische Wirkung des organischen Materials oder die Körpertemperatur, unmittelbar detektiert. Es ist dann nicht erforderlich, dass die Person eine Einrichtung zu ihrer Detektion (wie z.B. eine
Markereinrichtung, einen Signaleber, einen Sender oder einen RFID-chip) mit sich führt, welche von der
Detektionseinrichtung detektierbar ist und so die Person mittelbar detektierbar ist.

Ein Kapazitätssensor kann beispielsweise derart ausgebildet sein, dass er Kapazitätsänderungen erkennt, welche aufgrund der dielektrischen Eigenschaften des Körpers der Person im Hygienebereich hervorgerufen werden. Ein solcher Sensor ermöglicht es außerdem, den Eintritt einer Person in beziehungsweise den Austritt einer Person aus dem Hygienebereich zu detektieren, auch wenn bereits mehrere Personen im Hygienebereich sind.

Andererseits kann auch vorteilhaft sein, wenn der Näherungssensor zur Erkennung einer von der zu detektierenden Person tragbaren Markereinrichtung ausgebildet ist. In diesem Fall wird die zu detektierende Person mittelbar über die mitgeführte Markereinrichtung erkannt. Denkbar ist beispielsweise, dass die Personen mit tragbaren RFID-Einrichtungen ("Radio-Frequency Identification" -Transponder) ausgestattet sind, welche von einem als zugeordneter RFID-Detektor ausgebildeten Näherungssensor erkennbar sind. Die von der Person mitgeführte Markereinrichtung kann beispielsweise komfortabel in eine ID-Kennkarte oder ein Namensschild integriert werden.

Eine besonders bevorzugte weitere Ausgestaltung ergibt sich dadurch, dass der Näherungssensor derart ausgebildet ist, dass der Eintritt einer Person in den Hygienebereich oder der Austritt einer Person aus dem Hygienebereich unabhängig vom Aufenthalt einer weiteren Person im Hygienebereich detektierbar ist - das heißt unabhängig davon detektierbar ist, ob sich eine weitere Person in dem Hygienebereich befindet.

Dadurch kann der Hygienebereich auch dann weiter überwacht werden, wenn sich bereits eine Person im Hygienebereich befindet. Dies ist im Klinikalltag von Vorteil, da oftmals eine Vielzahl von Personen gleichzeitig Tätigkeiten in einem Hygienebereich, beispielsweise an einem Krankenbett, ausüben müssen. Durch die genannte Ausgestaltung wird auch eine Kontamination durch eine zu einer bereits im Hygienebereich befindlichen Person hinzukommend weiteren Person vermieden.

Ein Näherungssensor mit den genannten Eigenschaften kann beispielsweise einen Kapazitätssensor umfassen, welcher die Kapazitätsänderungen aufgrund einer in den Hygienebereich eintretenden beziehungsweise einer den Hygienebereich verlassenden Person detektiert. Eine Kapazitätsänderung kann grundsätzlich weitgehend unabhängig von der Anzahl der sich bereits im Hygienebereich aufhaltenden Personen detektiert werden. Ein Infrarotsensor kann die durch eine zusätzliche Person gebildete Wärmequelle weitgehend unabhängig von bereits vorhandenen Personen erkennen.

Eine vorteilhafte Ausgestaltung ergibt sich auch dadurch, dass der Näherungssensor derart ausgebildet ist, dass die Anzahl der sich im Hygienebereich befindlichen Personen detektierbar ist. Ist direkt die Anzahl der Personen im Hygienebereich bekannt, so kann ein Eintritt oder Austritt einer Person über der Änderung der Anzahl erkannt werden.

Zur weiteren Ausgestaltung des Systems ist vorgesehen, dass der Näherungssensor zum Zusammenwirken mit der Warneinrichtung eine Kontakteinrichtung zum Aufbau einer drahtlosen Kommunikationsverbindung, insbesondere Funkverbindung oder Infrarot-Verbindung, aufweist.

Eine drahtlose Verbindung zum Näherungssensor ermöglicht es, den Näherungssensor je nach Bedarf zu platzieren beziehungsweise seine Anordnung zu verändern. Eine Neuverkabelung kann dabei vermieden werden. Dadurch wird ein flexibler Einsatz des Systems für verschiedene räumliche Gegebenheiten ermöglicht. Außerdem ist ein Austausch des Näherungssensors zu Reinigungs- oder Wartungszwecken komfortabel möglich. Auch ein kompletter Austausch des Näherungssensors ist ohne aufwändige Umbaumaßnahme möglich. Dies ist beispielsweise dann vorteilhaft, wenn der Näherungssensor als steriles Austauschteil zur einmaligen Verwendung ausgebildet ist. Im Klinikbetrieb kommen solche Einmalprodukte oftmals zum Einsatz, wenn ein direkter Kontakt mit Patienten erfolgt, beispielsweise wenn der Näherungssensor unmittelbar an einem Krankenbett angeordnet ist.

Die Kommunikationsverbindung erfolgt beispielsweise über eine gängige Bluetooth-Verbindung, welche günstig realisierbar ist. Die Ausstattung des Systems mit Bluetooth-Kommunikationsmitteln ermöglicht außerdem einen störungsfreien Betrieb einer Vielzahl von lösungsgemäßen Systemen in unmittelbarer Nähe zueinander.

Denkbar ist jedoch auch, dass die genannte Kontakteinrichtung als Teil einer Steckverbindung zur Herstellung einer verdrahteten Verbindung zum Näherungssensor ausgebildet ist. Eine solche Kontakteinrichtung kann einfach und kostengünstig realisiert werden.

In jedem Fall muss die Verbindung zwischen dem Näherungssensor und der Warneinrichtung nicht direkt erfolgen. Gefordert ist im weiten Sinne ein Zusammenwirken des Näherungssensors mit der Warneinrichtung. Dies kann auch mittelbar, beispielsweise über eine Kontrolleinrichtung oder zwischengeschaltete Vorrichtungen erfolgen, wie weiter unten erläutert.

Vorteilhaft ist außerdem, wenn der Näherungssensor Befestigungsmittel zur lösbaren Befestigung innerhalb des Hygienebereichs aufweist. Insbesondere können solche Befestigungsmittel dazu dienen, den Näherungssensor an einem Krankenbett anzuordnen. Dadurch ist es möglich, den Näherungssensor als leicht austauschbares Teil auszubilden, wodurch beispielsweise auf komfortable Weise eine Reinigung oder Reparatur ermöglicht wird.

Der Näherungssensor kann dann beispielsweise als separates Austauschteil, insbesondere als Einweg-Artikel, vertrieben werden. Als Befestigungsmittel können beispielsweise Klebeabschnitte oder Klebestreifen vorgesehen sein, welche an einem Halteabschnitt des Näherungssensors angeordnet sind. Denkbar ist auch, dass der Näherungssensor einen Teil einer Klettverschluss-Verbindung aufweist, wobei im Hygienebereich, beispielsweise an einem Krankenbett, der zugeordnete andere Teil der Klettverschluss-Verbindung vorgesehen ist. Auch mechanische Klemm-Verbindungen zur Befestigung des Näherungssensors können vorteilhaft sein, da dann auf unerwünschte chemische Bestandteile in Klebeverbindungen oder auf Klettverschluss-Verbindungen, an welchen sich Verunreinigungen ansammelt könnten, verzichtet werden kann.

Die Reinigungseinrichtung ist vorteilhafterweise als Handwascheinrichtung, Handwaschbecken, Desinfektionsmittelspender ausgebildet. Die

Reinigungseinrichtung kann jedoch auch andere Reinigungs-Mechanismen nutzen, beispielsweise kann die Reinigungseinrichtung als UV-Strahler oder als Ultraschall-Reinigungsbad ausgebildet sein. Je nach Bedarf kann hier auch auf in einem Klinikbetrieb bereits bestehende
Reinigungseinrichtungen zurückgegriffen werden.

Zur weiteren Ausgestaltung weist die Reinigungseinrichtung einen Betätigungssensor zum Erkennen der Betätigung der Reinigungseinrichtung auf. Die Wirkung des Sensors soll nicht einschränkend auf beispielsweise berührungsloses Erkennen einer Betätigung verstanden werden. Denkbar ist auch ein Kontaktschalter, welcher derart an der Reinigungseinrichtung angeordnet ist, dass der Kontaktschalter bei Betätigung der Reinigungseinrichtung ebenfalls betätigt wird. Unabhängig von seiner Ausgestaltung kann der Betätigungssensor an oder in eine im Klinikbetrieb bereits bestehende Reinigungsanlage eingebaut werden, wodurch auf kostengünstige Weise eine Reinigungseinrichtung im Sinne der Erfindung bereitgestellt werden kann.

Eine weitere Ausgestaltung der Erfindung ergibt sich dadurch, dass eine Kontrolleinrichtung vorgesehen ist, über welche die Reinigungseinrichtung, der Näherungssensor und die Warneinrichtung sowie eine etwaige Warnunterdrückungseinrichtung zusammenwirken. Die genannten Mittel und Einrichtungen sind damit sämtlich mit der Kontrolleinrichtung verbunden und wirken mittelbar zusammen. Insbesondere ist denkbar, dass in der Kontrolleinrichtung Funktionsparameter des lösungsgemäßen Systems wie beispielsweise die erläuterte Wartedauer, die Durchgangszeitdauer oder die Stummzeitdauer vorgebbar sind. Dadurch kann das System durch entsprechende Konfiguration der Kontrolleinrichtung sehr flexibel eingesetzt werden.

Vorteilhaft ist auch, wenn die Warneinrichtung zur Abgabe eines akustischen und/oder optischen Warnsignals ausgebildet ist. Hierzu kann die Warneinrichtung eine Leuchteinrichtung zur Abgabe eines Leuchtsignals und/oder einen Signalgeber zur Abgabe eines Signaltons aufweisen. Je nach Umfeld kann es jedoch auch vorteilhaft sein, eine geräuschlose und/oder nicht leuchtende Warneinrichtung einzusetzen, beispielsweise eine Einrichtung zur Abgabe eines Vibrationsalarmes.

Eine weitere Ausgestaltung ergibt sich auch dadurch, dass eine Photovoltaik-Einrichtung zur Energieversorgung des Näherungssensors und/oder Warneinrichtung und/oder der Reinigungseinrichtung und/oder einer etwaigen Kontrolleinrichtung vorgesehen ist. Insbesondere ist die Photovoltaik-Einrichtung, beispielsweise Solarzelle, zur Energieversorgung des Näherungssensors an dem Näherungssensor angeordnet beziehungsweise dort befestigt. Vorteilhafterweise sind zusätzlich ein oder mehrere Energiespeicher, insbesondere Akkumulatoren, dem Näherungssensor und/oder der Warneinrichtung und/oder der Reinigungseinrichtung zugeordnet, insbesondere gemeinsam mit der jeweiligen Photovoltaik-Einrichtung, angeordnet. Die netzunabhängige Energieversorgung ist insbesondere vorteilhaft, wenn der Näherungssensor als Austauschteil oder Einweg-Artikel ausgebildet ist und über eine drahtlose Kommunikationsverbindung in das System eingebunden ist.

Die eingangs gestellte Aufgabe wird außerdem durch eine Sensoreinrichtung gelöst, welche zur Verwendung mit einem erfindungsgemäßen System wie vorstehend erläutert vorgesehen ist, wobei der Näherungssensor an der Sensoreinrichtung angeordnet ist.

Die Sensoreinrichtung kann zusätzlich auch weitere, mit dem Näherungssensor zusammenwirkende Teile des vorstehend beschriebenen Systems umfassen, beispielsweise eine Kontakteinrichtung zum Aufbau einer drahtlosen Kommunikationsverbindung zum Näherungssensor. Die den Näherungssensor umfassende Sensoreinrichtung ist in dem beschriebenen System insbesondere als auswechselbares Austauschteil, vorteilhafterweise auch als Einwegartikel, verwendbar. Sämtliche Merkmale zur Ausgestaltung des Systems, welche vorstehend im Zusammenhang mit dem Näherungssensor beschrieben wurden, können auch zur vorteilhaften Ausgestaltung der Sensoreinrichtung dienen, an welcher der Näherungssensor angeordnet ist.

Insbesondere ist vorteilhaft, wenn die Sensoreinrichtung einen flexiblen, bandartigen Trägerstreifen mit einer Oberseite und einer Unterseite aufweist, wobei der Näherungssensor an der Oberseite des Trägerstreifens angeordnet ist und wobei an der Unterseite des Trägerstreifens Befestigungsmittel zur lösbaren Befestigung der Sensoreinrichtung innerhalb des Hygienebereiches, insbesondere an einem Krankenbett, angeordnet sind. Eine derartige flexible, bandartige Sensoreinrichtung kann auf einfache Weise an verschiedenen Gegenständen im Hygienebereich und in verschiedenen Positionen angeordnet werden. Die lösbaren Befestigungsmittel ermöglichen es, die Sensoreinrichtung als Ganzes auszutauschen.

Eine vorteilhafte Ausgestaltung ergibt sich auch dadurch, dass der Näherungssensor beziehungsweise die genannte Sensoreinrichtung zur Befestigung an einem Krankenbett ausgebildet ist, insbesondere an einer Langseite des Krankenbettes, von der aus Zugriff auf das Krankenbett erfolgen kann. Weist die Sensoreinrichtung wie beschrieben einen flexiblen, bandartigen Trägerstreifen auf, so kann der Trägerstreifen beispielsweise entlang der genannten Langseite des Krankenbettes, oder entlang eines Bett-Gitters zur Sicherung des Patienten im Krankenbett verlegt werden.

Die eingangs gestellte Aufgabe wird auch durch ein Verfahren zur Unterstützung beziehungsweise zur Kontrolle der Reinhaltung eines von Personen betretbaren Hygienebereiches gelöst, bei welchem insbesondere ein System wie vorstehend beschrieben Verwendung findet, wobei ein Eintritt einer Person in den Hygienebereich oder ein Austritt einer Person aus dem Hygienebereich detektiert wird, und ein Warnsignal nach Detektion eines Eintritts einer Person in den Hygienebereich oder eines Austritts einer Person aus dem Hygienebereich ausgelöst wird, insbesondere nach einer Wartedauer nach der Detektion ausgelöst wird, wobei die Auslösung des Warnsignals durch Betätigung einer Reinigungseinrichtung zur Reinigung einer Hand einer Person unterdrückt wird.

Durch dieses Verfahren werden die den Hygienebereich betretenden oder verlassenden Personen angehalten, die Reinigungseinrichtung zu betätigen, insbesondere ihre Hände zu waschen. Dadurch können die bereits im Zusammenhang mit dem erfindungsgemäßen System erläuterten Vorteile erzielt werden.

Dementsprechend ergeben sich vorteilhafte Ausgestaltungen des Verfahrens durch die bereits im Zusammenhang mit dem lösungsgemäßen System beschriebenen Merkmale, sowie durch die im Zusammenhang mit diesen Merkmalen beschriebenen Funktionen und Effekte.

Beispielsweise ist es vorteilhaft, wenn innerhalb einer Durchgangszeitdauer nach Betätigung der Reinigungseinrichtung die Auslösung des Warnsignals unterdrückt wird beziehungsweise unterbleibt, das heißt die Auslösung bei Detektion eines Eintritts oder Austritts innerhalb der Durchgangszeitdauer nicht erfolgt.

Weitere Einzelheiten und vorteilhafte Ausgestaltungen der Erfindung sind der nachfolgenden Beschreibung zu entnehmen, anhand derer die Figuren näher beschrieben und erläutert sind.

Es zeigen:
- Figur 1: schematische Darstellung eines erfindungsgemäßen Systems;
- Figur 2: schematische Darstellung einer weiteren Ausführungsform eines erfindungsgemäßen Systems; und
- Figur 3: schematische Darstellung zur Erläuterung des erfindungsgemäßen Verfahrens beziehungsweise der Funktion des erfindungsgemäßen Systems.

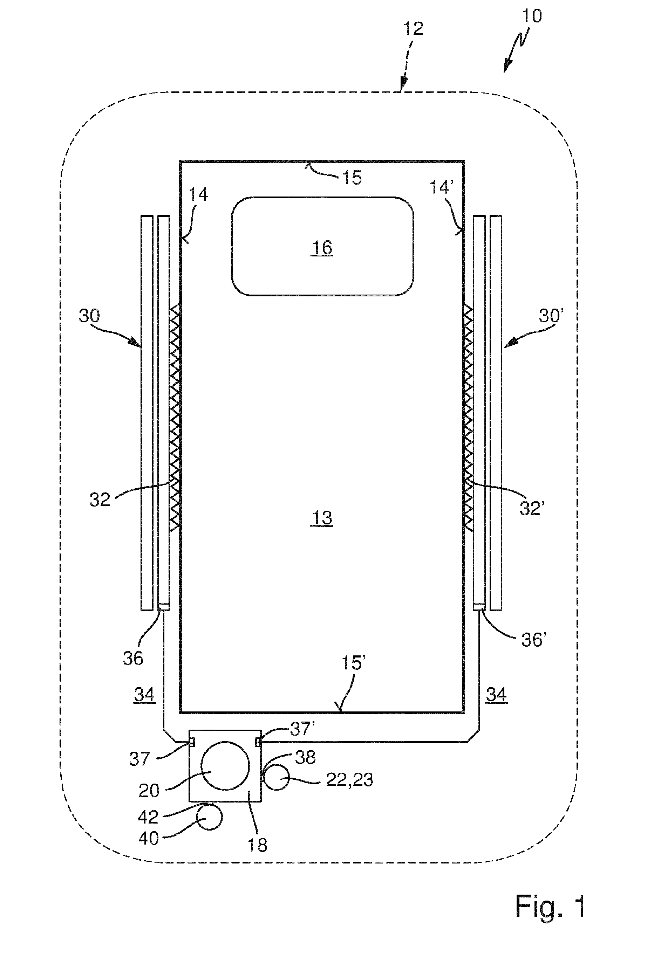

In den Figuren 1 bis 3 sind zur besseren Übersichtlichkeit einander funktional entsprechende Bauteile oder Merkmale durchgehend mit denselben Bezugszeichen versehen.

Die Figur 1 zeigt ein System 10 (bzw. eine Vorrichtung 10), welches zur Unterstützung der Reinhaltung beziehungsweise zur Kontrolle der Reinhaltung eines von nicht dargestellten Personen betretbaren beziehungsweise begehbaren Hygienebereiches 12 vorgesehen ist.

Der Hygienebereich 12 umfasst ein Krankenbett 13 mit zwei einander gegenüberliegenden Langseiten 14, 14', zwei Schmalseiten 15, 15' sowie einem Kopfteil 16 für einen nicht dargestellten Patienten. Ein Zugriff auf den Patienten zu Behandlungszwecken erfolgt insbesondere über die Langseiten 14, 14'. Medizinisches Personal nähert sich daher bevorzugt im Bereich der Langseiten 14, 14' dem Krankenbett 13 an.

Mit Hilfe des Systems 10 soll insbesondere verhindert werden, dass Personen, welche den Hygienebereich 12 betreten, auf das Krankenbett 13 beziehungsweise auf den darauf liegenden Patienten zugreifen, ohne sich vorher die Hände zu waschen. Ebenso soll verhindert werden, dass nach Berührung des Patienten, beispielsweise bei einer Behandlung, eine Person den Hygienebereich 12 verlässt, ohne sich die Hände zu waschen.

Hierzu ist eine beispielsweise als Handwaschbecken ausgebildete Reinigungseinrichtung 18 vorgesehen, welche im Bereich der unteren Schmalseite 15' des Krankenbettes 13 angeordnet ist. Ein Zugriff auf das Krankenbett 13 über die Langseiten 14, 14' wird daher nicht durch die Reinigungseinrichtung 18 behindert.

Die Reinigungseinrichtung 18 weist einen in der Figur 1 nur skizzierten Betätigungssensor 20 auf, mit welchem eine Betätigung der Reinigungseinrichtung 18 erkennbar ist.

Vorgesehen ist außerdem eine Warneinrichtung 22, welche im Bereich der Reinigungseinrichtung 18 angeordnet ist. Zur Abgabe eines Warnsignals weist die Warneinrichtung 22 beispielsweise eine Leuchteinrichtung 23 auf, mit welcher ein Leuchtsignal abgegeben werden kann.

Zur Detektion eines Eintritts einer Person in den Hygienebereich 12 beziehungsweise eines Austritts einer Person aus dem Hygienebereich 12 sind bei dem System 10 zwei Näherungssensoren 30, 30' vorgesehen. Die Näherungssensoren 30, 30' sind als sich bandartig erstreckende Kapazitätssensoren ausgebildet, mittels welchen die Annäherung einer Person durch Änderung einer von dem jeweiligen Kapazitätssensor bestimmbaren Kapazität detektierbar ist.

Der als Kapazitätssensor ausgebildete Näherungssensor 30 ist dabei sich entlang der Langseite 14 des Krankenbettes 13 erstreckend an dem Krankenbett 13 angeordnet. Entsprechend ist der Näherungssensor 30' sich entlang der Langseite 14' erstreckend am Krankenbett 13 angeordnet. Hierzu hat der Näherungssensor 30 ein als Klebeabschnitt ausgebildetes Befestigungsmittel 32, mittels welchem der Näherungssensor 30 lösbar an der Langseite 14 angeklebt und wieder entfernt werden kann. Entsprechend ist für den Näherungssensor 30' ein als Klebeabschnitt ausgebildetes Befestigungsmittel 32' vorgesehen.

Da die Näherungssensoren 30, 30' sich entlang der Langseiten 14, 14' des Krankenbettes 13 erstreckend angeordnet sind, kann mit den Näherungssensoren 30, 30' zuverlässig eine Annäherung einer Person oder ein Zugriff auf das Krankenbett 13 detektiert werden.

Der Näherungssensor 30 beziehungsweise der Näherungssensor 30' wirkt mit der Reinigungseinrichtung 18 und mit der
Warneinrichtung 22 über eine drahtlose
Kommunikationsverbindung 34 zusammen. Die
Kommunikationsverbindung 34 ist in der Figur 1 mit einer durchgezogenen Linie zwischen den Näherungssensoren 30, 30' und der Reinigungseinrichtung 18 symbolisiert. Die Näherungssensoren 30, 30' wirken dabei auch mit der Warneinrichtung 22 zusammen, wobei diese Zusammenwirkung mittelbar über die Reinigungseinrichtung 18 erfolgt, an welcher die Warneinrichtung 22 angeordnet ist.

Die genannte Kommunikationsverbindung 34 kann beispielsweise über eine Bluetooth-Verbindung realisiert werden. Stattdessen ist selbstverständlich auch eine Verdrahtung der Näherungssensoren 30, 30' mit der Reinigungseinrichtung 18 beziehungsweise mit der Warneinrichtung 22 denkbar.

Zum Aufbau der Kommunikationsverbindung 34 weist der Näherungssensor 30 beziehungsweise der Näherungssensor 30' jeweils eine Kontakteinrichtung 36 beziehungsweise 36' auf, welche beispielsweise als Funk-Sendeempfänger beziehungsweise Infrarot-Sendeempfänger ausgebildet ist. Entsprechend hierzu sind an der Reinigungseinrichtung 18 zugeordnete Kontakteinrichtungen 37, 37' zur Zusammenwirkung mit den Näherungssensoren 30, 30' vorgesehen. Auch die Warneinrichtung 22 weist eine Kontakteinrichtung 38 auf, mittels welcher eine Zusammenwirkung mit den Näherungssensoren 30, 30' beziehungsweise mit der Reinigungseinrichtung 18 erfolgen kann.

Bei dem System 10 ist ferner eine Warnunterdrückungseinrichtung 40 vorgesehen, welche ebenfalls an bzw. im Bereich der Reinigungseinrichtung 18 angeordnet ist. Die Warnunterdrückungseinrichtung 40 ist beispielsweise als Druckschalter ausgebildet. Auch für die
Warnunterdrückungseinrichtung 40 ist wiederum eine in der Figur 1 nur skizzierte Kontakteinrichtung 42 vorgesehen, über welche die Warnunterdrückungseinrichtung 40 mit der Warneinrichtung 22 zusammenwirken kann.

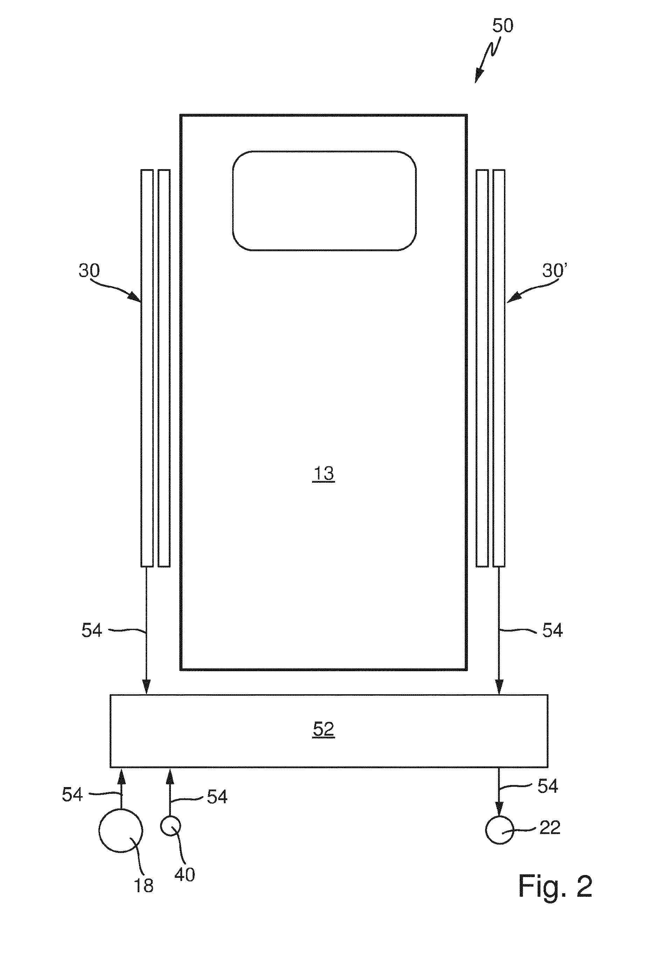

Bei dem in der Figur 2 schematisch dargestellten System 50 erfolgt die Zusammenwirkung zwischen dem Näherungssensor 30 beziehungsweise 30' und der Warneinrichtung 22 beziehungsweise zwischen der Reinigungseinrichtung 18 und der Warneinrichtung 22 sowie zwischen der Warnunterdrückungseinrichtung 40 und der Warneinrichtung 22 über eine Kontrolleinrichtung 52.

Hierzu sind der Näherungssensor 30 beziehungsweise 30', die Reinigungseinrichtung 18, die Warneinrichtung 22 sowie die Warnunterdrückungseinrichtung 40 jeweils über eine in der Figur 2 angedeutete Kommunikationsverbindung 54 mit der Kontrolleinrichtung 52 verbunden, und weisen hierfür entsprechende (nicht dargestellte) Kontakteinrichtungen auf.

Mit Hilfe der Kontrolleinrichtung 52 kann die Art und Weise der Zusammenwirkung zwischen den verschiedenen Bauteilen des Systems 50 beeinflusst werden. Hierzu ist die
Kontrolleinrichtung 52 entsprechend programmierbar.

Neben den genannten Paarungen zur Zusammenwirkung (Näherungssensor 30, 30' mit Warneinrichtung 22, Reinigungseinrichtung 18 mit Warneinrichtung 22, Warnunterdrückung 40 mit Warneinrichtung 22) können über die Kontrolleinrichtung 52 auch weitere Arten der Zusammenwirkung erfolgen. Beispielsweise ist denkbar, dass die Warnunterdrückungseinrichtung 40 mit den Näherungssensoren 30, 30' zusammenwirkt, beispielsweise um bei Betätigung der Warnunterdrückungseinrichtung 40 eine Detektion durch die Näherungssensoren 30, 30' zu unterdrücken. Ebenso ist eine Zusammenwirkung zwischen der Reinigungseinrichtung 18 und den Näherungssensoren 30, 30' denkbar. Dies gilt auch bei dem in der Figur 1 dargestellten System 10, da dort die genannten Bauteile grundsätzlich alle über die Kommunikationsverbindung 34 miteinander zusammenwirken können.

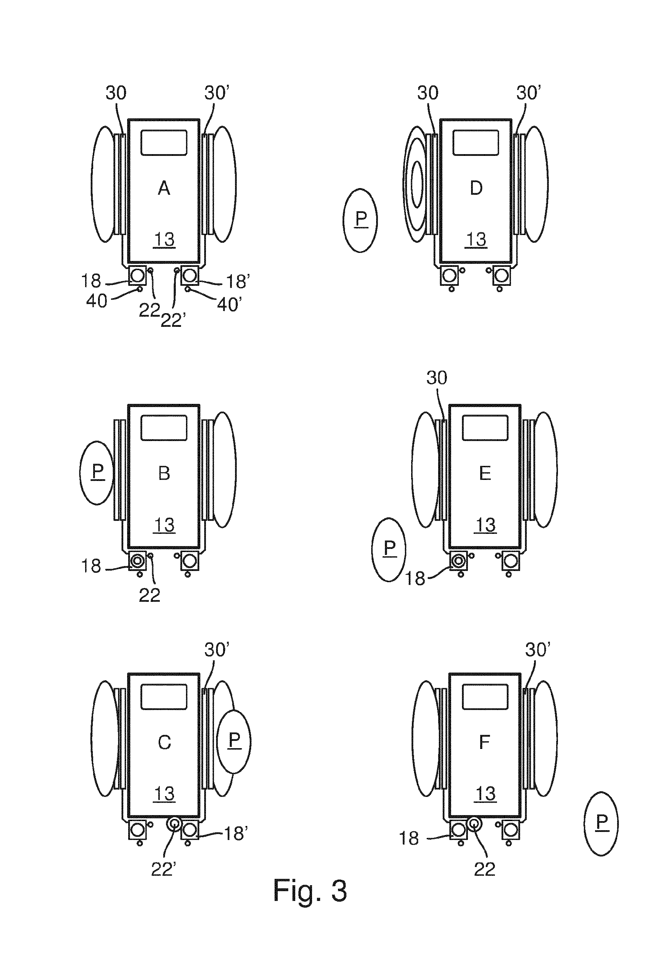

Die Figur 3 zeigt zur Erläuterung der Funktionsweise der Erfindung, insbesondere der Systeme 10 und 30 gemäß Figuren 1 und 2, sechs mit "A" bis "F" bezeichnete Funktionszustände.

Die Funktionszustände werden dabei anhand eines Aufbaus erläutert, welcher von dem System 10 gemäß Figur 1 geringfügig abweicht: Jedem der Näherungssensoren 30, 30' ist jeweils eine eigene Reinigungseinrichtung 18 beziehungsweise 18' sowie eine eigene Warneinrichtung 22 beziehungsweise 22' zugeordnet. Der Näherungssensor 30 wirkt dabei nur mit der
Reinigungseinrichtung 18 und der Warneinrichtung 22 zusammen. Der Näherungssensor 30' wirkt entsprechend nur mit der Reinigungseinrichtung 18' und der Warneinrichtung 22' zusammen. Entsprechend ist jeweils eine Warnunterdrückungseinrichtung 40 beziehungsweise 40' vorgesehen.

Bei dem in der Figur 3 skizzierten Krankenbett 13 sind insofern zwei voneinander unabhängige erfindungsgemäße Systeme vorgesehen, welche sich von dem System 10 gemäß Figur 1 im Wesentlichen dadurch unterscheiden, dass jeweils nur ein Näherungssensor 30 beziehungsweise 30' Verwendung findet.

Der mit "A" bezeichnete Funktionszustand zeigt ein erfindungsgemäßes System in einem aktivierten
Überwachungszustand. Keiner der Näherungssensoren 30, 30' detektiert in der zu "A" dargestellten Situation ein Annähern oder Entfernen einer Person. Der Näherungssensor 30 wirkt jedoch derart mit der Warneinrichtung 22 zusammen, dass die Warneinrichtung 22 ein Warnsignal auslöst, wenn der Näherungssensor 30 eine Annäherung beziehungsweise ein Entfernen einer Person detektiert. Entsprechendes gilt für den Näherungssensor 30 und die Warneinrichtung 22'.

Bei den weiteren mit den Bezugszeichen "B" bis "F" versehenen schematischen Darstellungen der Figur 3 wurde zur besseren Übersichtlichkeit größtenteils auf Bezugszeichen verzichtet, da es sich jeweils um dieselbe Anordnung wie in der schematischen Darstellung zu "A" handelt.

Bei der Darstellung zu "B" hat sich eine Person dem Krankenbett 13 genähert, wobei die Person schematisch dargestellt ist und mit dem Bezugszeichen "P" bezeichnet ist. Vor der Annäherung an das Krankenbett 13 hat die Person P die Reinigungseinrichtung 18 betätigt. Die Reinigungseinrichtung 18 wirkt dabei derart mit der Warneinrichtung 22 zusammen, dass nach Betätigung der Reinigungseinrichtung innerhalb einer sich an die Betätigung anschließenden Durchgangszeitdauer die Auslösung des Warnsignals unterbleibt, auch wenn der Näherungssensor 30 die Annäherung der Person P detektiert. Diese Durchgangszeitdauer beträgt beispielsweise zehn Sekunden. Nach Betätigung der Reinigungseinrichtung 18 hat die Person P somit die Möglichkeit, sich dem Krankenbett 13 so anzunähern, wie es die Darstellung zu "B" zeigt.

Die Darstellung zu "C" zeigt hingegen die Situation, bei der sich die Person P dem Näherungssensor 30' angenähert hat, ohne vorher die zugeordnete Reinigungseinrichtung 18' zu betätigen. Da der Näherungssensor 30' mit der Warneinrichtung 22' zusammenwirkt, wird bei Detektion der Person P demnach ein Warnsignal ausgelöst. Das ausgelöste Warnsignal ist in der Darstellung zu "C" durch Hervorhebung der Warneinrichtung 22' angedeutet.

Für eine bessere Nutzbarkeit des erfindungsgemäßen Systems kann jedoch vorgesehen sein, dass der Näherungssensor 30' derart mit der Warneinrichtung 22' zusammenwirkt, dass die Auslösung des Warnsignals bei Detektion der Annäherung der Person P ohne vorherige Betätigung der Reinigungseinrichtung 18' dennoch erst dann erfolgt, wenn nach der Detektion der Annäherung der Person P auch keine Betätigung der Reinigungseinrichtung 18' innerhalb einer Wartedauer erfolgt. Diese Wartedauer kann beispielsweise im Bereich von zehn Sekunden gewählt werden. Dadurch wird der Person P die Möglichkeit gegeben, einen unterbliebenen Waschvorgang mit der Reinigungseinrichtung 18' nachzuholen, und so möglicherweise noch eine Kontamination des Krankenbettes 13 zu verhindern.

Wie aus der Darstellung "D" hervorgeht, kann mit den Näherungssensoren 30 beziehungsweise 30' auch detektiert werden, dass sich eine Person P von dem Krankenbett 13 entfernt, die sich bisher am Krankenbett 13 aufgehalten hat.

Entfernt sich die Person P vom Krankenbett 13 nach einer vorausgegangenen Betätigung der Reinigungseinrichtung 18, so unterbleibt eine Auslösung eines Warnsignals, obwohl die Entfernung der Person P von dem Näherungssensor 30 detektiert wird.

Die Auslösung des Warnsignals unterbleibt ferner auch dann, wenn die Person P noch innerhalb einer Wartedauer von cirka zehn Sekunden nach Detektion ihres Entfernens durch den Näherungssensor 30 die Reinigungseinrichtung 18 betätigt. Umgekehrt wird ein Warnsignal jedoch auch nach Betätigung der Reinigungseinrichtung 18 ausgelöst, wenn die Detektion des Entfernens der Person P durch den Näherungssensor 30 erst nach einer Durchgangszeitdauer von cirka zehn Sekunden nach Betätigung der Reinigungseinrichtung 18 erfolgt.

Die Darstellung zu "F" in Figur 3 zeigt einen solchen Fall, bei dem durch die entfernende Person P ein Warnsignal ausgelöst wurde, wie durch die hervorgehobene Darstellung der Warneinrichtung 22 angedeutet ist.

Nach Auslösung eines Warnsignals, wie in den Darstellungen "C" und "F" angedeutet, hält das abgegebene Warnsignal für eine Warndauer von beispielsweise dreißig Sekunden an.

Unabhängig von der genauen Ausgestaltung des Systems, insbesondere also auch bei dem System 10 gemäß Figur 1 und dem System 50 gemäß Figur 2, kann vorgesehen sein, dass die Reinigungseinrichtung 18 (beziehungsweise 18') derart mit dem jeweiligen Näherungssensor 30 (beziehungsweise 30') zusammenwirkt, dass nach Auslösung eines Warnsignals das anhaltende Warnsignal abgeschaltet wird, wenn eine Betätigung der Reinigungseinrichtung 18 (beziehungsweise 18') erfolgt.

Die Auslösung eines Warnsignals bei Annäherung (Darstellung "C") beziehungsweise bei Entfernung (Darstellung "F") einer Person P kann außerdem auch dadurch unterbunden werden, dass die Person P anstelle der Reinigungseinrichtung 18' (im Falle "C") beziehungsweise 18 (im Falle "F") die Warnunterdrückungseinrichtung 40' (im Falle "C") beziehungsweise 40 (im Falle "F") betätigt. Damit kann sich die Person P dem Krankenbett 13 annähern beziehungsweise sich von diesem entfernen, ohne dass ein Reinigungsvorgang (Händewaschen) erfolgt und dennoch kein Warnsignal ausgelöst wird. Dies kann beispielsweise erwünscht sein, wenn lediglich eine Sichtkontrolle eines Patienten auf dem Krankenbett 13 beabsichtigt ist, bei der eine Verunreinigung oder Kontamination nicht droht.

## Patentansprüche

1. System (10, 50) zur Unterstützung der Reinhaltung eines von Personen betretbaren Hygienebereiches (12),
- mit einer Reinigungseinrichtung (18, 18'), die zur Reinigung einer Hand einer Person betätigbar ist,
- mit einer Warneinrichtung (22, 22') zum Auslösen eines Warnsignals,
- mit einem Näherungssensor (30, 30') zur Detektion eines Eintritts einer Person in den Hygienebereich (12) oder eines Austritts einer Person aus dem Hygienebereich (12), wobei die Warneinrichtung (22,22') derart mit dem Näherungssensor (30, 30') zusammenwirkt, dass das Warnsignal aufgrund einer Detektion eines Eintritts oder Austritts auslösbar ist,
und wobei die Warneinrichtung (22, 22') derart mit der Reinigungseinrichtung (18, 18') zusammenwirkt, dass die Auslösung des Warnsignals durch die Betätigung der Reinigungseinrichtung (18, 18') unterdrückbar ist.

2. System (10, 50) nach Anspruch 1, **dadurch gekennzeichnet, dass** die Reinigungseinrichtung (18, 18') und/oder der Näherungssensor (30, 30') innerhalb des Hygienebereiches (12) angeordnet ist.

3. System (10, 50) nach Anspruch 1 oder 2, **dadurch gekennzeichnet, dass** die Warneinrichtung (22, 22') derart mit dem Näherungssensor (30, 30') zusammenwirkt, dass das Warnsignal bei Detektion eines Eintritts oder Austritts erst nach einer Wartedauer nach der Detektion auslösbar ist.

4. System (10. 50) nach einem der vorhergehenden Ansprüche, **dadurch gekennzeichnet, dass** die Reinigungseinrichtung (18, 18') derart mit der Warneinrichtung (22, 22') zusammenwirkt, dass aufgrund einer Betätigung der Reinigungseinrichtung (18, 18') die Auslösung des Warnsignals unmittelbar unterdrückt wird, insbesondere die Auslösung des Warnsignals durch Betätigung der Reinigungseinrichtung (18, 18') immer unterdrückt wird.

5. System (10, 50) nach einem der vorhergehenden Ansprüche, **dadurch gekennzeichnet, dass** die Warneinrichtung (22, 22`) derart mit der Reinigungseinrichtung (18, 18`) zusammenwirkt, dass innerhalb einer Durchgangszeitdauer nach Betätigung der Reinigungseinrichtung (18, 18') die Auslösung des Warnsignals unterbleibt.

6. System (10, 50) nach einem der vorhergehenden Ansprüche, **dadurch gekennzeichnet, dass** eine Warnunterdrückungseinrichtung (40, 40') vorgesehen ist, die zur Unterdrückung der Auslösung des Warnsignals unabhängig von der Betätigung der Reinigungseinrichtung (18, 18') betätigbar ist.

7. System (10, 50) nach dem vorhergehenden Anspruch, **dadurch gekennzeichnet, dass** die Warnunterdrückungseinrichtung (40, 40') derart mit der Warneinrichtung (22, 22') zusammenwirkt, dass die Auslösung des Warnsignals nach Betätigung der Warnunterdrückungseinrichtung (40, 40') für eine Stummzeitdauer unterdrückbar ist.

8. System (10, 50) nach einem der vorhergehenden Ansprüche, **dadurch gekennzeichnet, dass** der Näherungssensor (30, 30') zur unmittelbaren Detektion einer Person ausgebildet ist, insbesondere derart ausgebildet ist, dass eine Person aufgrund von physikalischen Eigenschaften des menschlichen Körpers detektierbar ist, insbesondere als Kapazitätssensor oder als optischer Sensor oder als Infrarotsensor zur Detektion einer Person ausgebildet ist.

9. System (10, 50) nach einem der vorhergehenden Ansprüche, **dadurch gekennzeichnet, dass** der Näherungssensor (30, 30') derart ausgebildet ist, dass der Eintritt einer Person in den Hygienebereich (12) oder der Austritt einer Person aus dem Hygienebereich (12) unabhängig davon detektierbar ist, ob sich eine weitere Person in dem Hygienebereich (12) befindet.

10. System (10) nach einem der vorhergehenden Ansprüche, **dadurch gekennzeichnet, dass** der Näherungssensor (30, 30') zum Zusammenwirken mit der Warneinrichtung (22) eine Kontakteinrichtung (36) zum Aufbau einer drahtlosen Kommunikationsverbindung (34), insbesondere Funkverbindung oder Infrarot-Verbindung, aufweist.

11. System nach einem der vorhergehenden Ansprüche, **dadurch gekennzeichnet, dass** der Näherungssensor (30, 30') Befestigungsmittel (32, 32') zur lösbaren Befestigung innerhalb des Hygienebereiches (12), insbesondere an einem Krankenbett (13), aufweist.

12. System (10) nach einem der vorhergehenden Ansprüche, **dadurch gekennzeichnet, dass** die Reinigungseinrichtung (18) einen Betätigungssensor (20) zum Erkennen einer Betätigung der Reinigungseinrichtung (18) aufweist.

13. System (10) nach einem der vorhergehenden Ansprüche, **dadurch gekennzeichnet, dass** die Warneinrichtung (22) zur Abgabe eines akustischen und/oder optischen Warnsignals ausgebildet ist.

14. Sensoreinrichtung zur Verwendung mit einem System nach einem der vorhergehenden Ansprüche, wobei der Näherungssensor an der Sensoreinrichtung angeordnet ist, wobei die Sensoreinrichtung einen flexiblen, bandartigen Trägerstreifen mit einer Oberseite und einer Unterseite aufweist, wobei der Näherungssensor an der Oberseite angeordnet ist und wobei an der Unterseite ein Befestigungsmittel zur lösbaren Befestigung innerhalb des Hygienebereiches, insbesondere an einem Krankenbett, angeordnet ist.

15. Verfahren zur Unterstützung der Reinhaltung eines von Personen betretbaren Hygienebereiches (12), insbesondere mit einem System nach einem der vorhergehenden Ansprüche, wobei
- ein Eintritt einer Person in den Hygienebereich (12) oder ein Austritt einer Person aus dem Hygienebereich (12) detektiert wird,
- ein Warnsignal nach Detektion eines Eintritts oder Austritts, insbesondere nach einer Wartedauer nach der Detektion, ausgelöst wird,
wobei die Auslösung des Warnsignals durch Betätigung einer Reinigungseinrichtung (18, 18') zur Reinigung einer Hand einer Person unterdrückt wird.
